# EUROPEAN PATENT APPLICATION

(11) **EP 0 935 956 A2**
(43) Date of publication of application: **18.08.1999**
(21) Application number: 99300786.3
(22) Date of filing: 03.02.1999
(51) Int. Cl.: A61G 13/00

(54) **Delivery device**

(30) Priority: 17.02.1998 JP 5001298
(71) Applicant: Igei, Seishun, Ginowan-shi, Okinawa (JP)
(72) Inventor: Igei, Seishun, Ginowan-shi, Okinawa (JP)
(74) Representative: Whalley, Kevin

(57) **Abstract**

A delivery device wherein the delivery of a baby is stimulated by sliding or oscillating a delivery table capable of colliding against a stopper so that the delivery can be easily and safely attained. A preferred embodiment of such a delivery device comprises a delivery table (2) slidably laid on a base (1), and a colliding part (6) for impacting against a stopper (4) optionally disposed on the base (1) or other position and formed at an end in a leg direction of the delivery table.

## Description

The present invention relates to a delivery table used for a delivery.

In order to attain or stimulate the delivery without suffering, it was hitherto customary for a nurse or a doctor to give a pregnant woman a physical stimulus by rubbing her with their hands, administer her an analgesic, give her an aesthetic treatment or give her an intravenous drip to promote the delivery. Further, training by an overland walking or an underwater walking or various trials such as an underwater childbirth have been performed.

An act of giving a physical stimulus as executed in the past causes a fairly big burden in time and labor to a hospital suffering from a lack of manpower. Further, the underwater childbirth has medical and hygienic uncertainty and it is difficult for everyone to adapt it.

Therefore, the present invention intends to stimulate the delivery through the impact by disposing a means for giving an impact to the delivery table on which the pregnant woman lies in.

For this reason, the delivery table is designed such that it is slid and hit against a stopper or it is directly given the impact by an impact means.

The present invention in a first aspect provides a delivery device which comprises a delivery table slidably laid on a base, and a colliding part for impacting against a stopper optionally disposed on the base or other position and formed at an end in a leg direction of the delivery table. Said stopper may comprise part of the delivery device itself, or alternatively may be disposed on a separate body such as an adjacent wall or floor or the like.

The delivery device of the invention preferably further comprises a handle used for sliding the delivery table, a step for placing a pregnant woman's leg for preventing the pregnant woman from slipping in the leg direction of the delivery table, and a belt for holding a body of the pregnant woman at a center portion thereof. A gripper for the pregnant woman to grasp may be disposed above the delivery table.

A roller may be disposed between the base and the delivery table.

The delivery device preferably further comprises means for tilting the delivery device. Specifically, the base or the delivery table may be tilted with known means.

The invention in a second aspect provides a delivery device, wherein a means for giving an impact from a leg direction to a head direction of a delivery table is disposed on a base on which the delivery table is laid.

The delivery device according to the second aspect of the invention preferably further comprises a step for placing a pregnant woman's leg for preventing the pregnant woman from slipping in the leg direction of the delivery table, and a belt for holding a body of the pregnant woman at a center portion thereof. A gripper for the pregnant woman to grasp may be disposed above the delivery table.

The said means for giving impact is preferably a rotary arm or an electric blow device.

The invention in a third aspect provides a delivery device, wherein a delivery table is arranged to oscillate in a longitudinal direction thereof and receives impact by colliding against a stopper for a pregnant woman's legs so as to stimulate delivery. Said stopper may comprise part of the delivery device itself, or alternatively may be disposed on a separate body such as an adjacent wall or floor or the like.

The delivery device according to the third aspect of the invention preferably further comprises a handle used for oscillating the delivery table, a step for placing a pregnant woman's legs for preventing the pregnant woman from slipping in the leg direction of the delivery table, and a belt for holding a body of the pregnant woman at a center portion thereof.

The invention will be further described, by way of example only, with reference to the accompanying drawings, in which:

Fig.1 is a schematic view, showing the structure of a slide type delivery table.

Fig.2 is a schematic view, showing the structure of a blow type delivery table.

Fig.3 is a schematic view, showing the structure of an oscillation type delivery table.

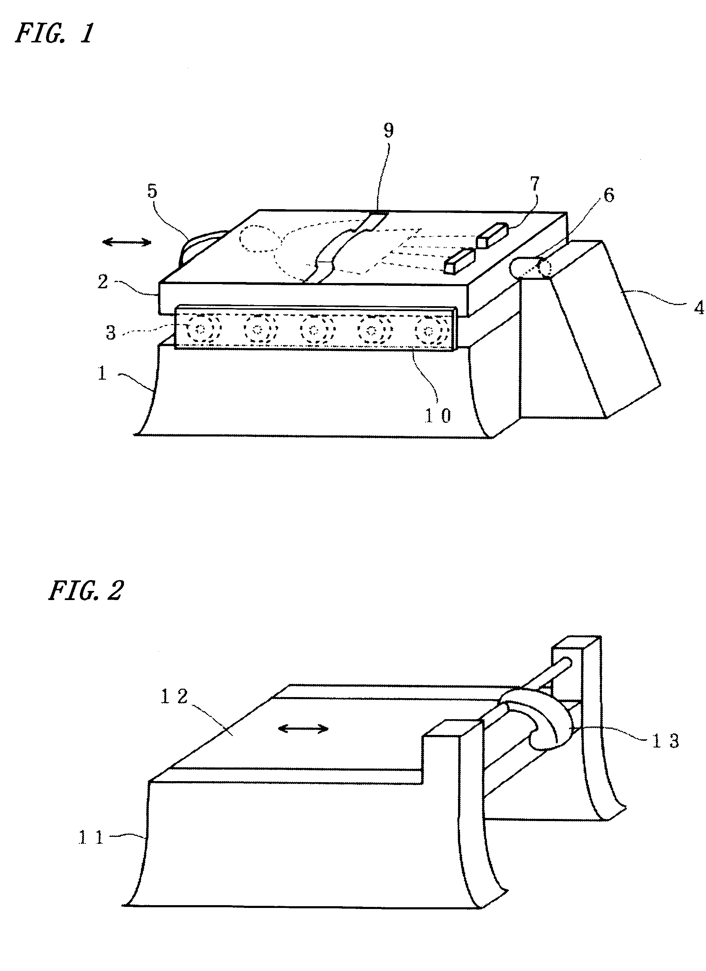

The present invention will be described with reference to an embodiment as shown in Fig.1.

A delivery table 2 for making a pregnant woman lie in is laid on a base 1,and a roller 3 is attached on the base 1 or to the delivery table 2 so as to smoothly slide the delivery table 2 on the base 1. This roller 3 may be replaced with a bearing ball or a sliderable member and the use of all is not limited, provided that the delivery table is constituted so as to be able to smoothly slide against the base 1. Further, when the base 1 or the delivery table 2 is formed so as to be able to tilt, it is advantageous in that it easily slides and can act by a weak force.

A stopper 4 is disposed in the leg direction of the base 1. This stopper may be disposed on a separate body such as an adjacent wall or floor or the like.

A handle 5 is attached with the delivery table 2 such that the delivery table when sliding can be easily moved. Further, a colliding part 6 hitting against the stopper 4 is projected in the leg end direction of the delivery table 2. When a tip of this colliding part is formed in the shape such as a semi-sphere, it is advantageous in that an impact effect can be fully worked out even if the collision against the stopper face is not straight.

Furthermore, a step 7 for placing the pregnant woman's leg and a belt 9 for fixing her body are provided on the delivery table 2 for preventing her slip at the time of impact.

The present invention is formed in such manner that, at the time of delivery, the base 1 or the delivery table 2 is preferably tilted, the handle 5 of the delivery table 2 is grasped and the delivery table is slightly slid upward and then let loose. As a result, the delivery table slips down by self-weight and the collision portion 6 collides against the stopper 4 and the impact is given to the pregnant woman fixed on the delivery table by the step 7 and the belt 9, thereby inducing the delivery. Since the pregnant woman is usually fixed under the condition opening her legs, her legs are more spread at the time of impact, thereby making it easy to discharge or take out a baby combined with its own weight.

At this time, since the colliding part 6 is formed in the shape of semi-sphere, even if there is a slight drift in the direction, the impact in the direction of the delivery table can be obtained. Further, since a space is formed between the stopper 4 and the delivery table 2 by projecting the colliding part 6, it also serves to prevent persons from getting hemmed in and harmed.

Thus, by repeating the action of the delivery table being slightly slid upward and let loose, the delivery can be simply stimulated.

Note that, though the description is made herein of the method for colliding the delivery table against the stopper by its own weight, the impact may be also given, of course, by colliding the delivery table against the stopper by man power.

Note also that, for controlling the sliding direction of the delivery table and preventing a fall in the lateral side, a guide wall 10 may be mounted to both side faces of the base or the delivery table.

Next, other example will be shown in Fig.2.

In this example, the delivery table is constituted such that the impact is given from a leg direction.

A delivery table 12 is sliderably attached to a base 11 and a blow means 13, for example, such as a rotary arm fixed to the base and the like is arranged the end part in its leg direction.

Other structures of the base 11 and the delivery table 12 are formed as described in said embodiment.

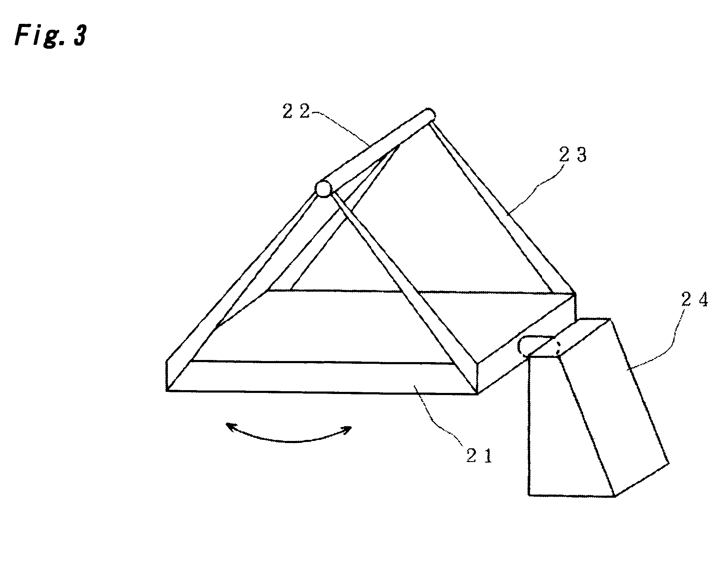

Further, another example will be shown in Fig.3.

A delivery table 21 is suspended from an arm 23 fixed to a shaft 22 and the like or a wire and the like and the delivery table is formed so as to be able to tilt in a swing pattern in the longitudinal direction, and the impact is given with the delivery table 21 hitting against a stopper 24.

The delivery table 21 is moved in her head direction and a space is formed between it and the stopper 24. Then, by making free, the delivery table constitutes to oscillate naturally and returns in the direction of the stopper to collide against the stopper.

Note that the impact may be given by pushing the delivery table in the direction of the stopper by manpower and making it collide against the stopper.

By only stimulating the delivery through the impact given to the delivery table on which the pregnant woman lying in, the delivery can be easily attained with a little treatment labor required for the doctor and the nurse for treatment such as an intravenous drip and the like. Hence, a safe delivery can be attained.

## Claims

1. A delivery device which comprises a delivery table (2) slidably laid on a base (1), and a colliding part (6) for impacting against a stopper (4) optionally disposed on the base (1) or other position and formed at an end in a leg direction of the delivery table.

2. A delivery device as claimed in claim 1, characterized by further comprising a handle (5) used for sliding the delivery table (2), a step (7) for placing a pregnant woman's leg for preventing the pregnant woman from slipping in the leg direction of the delivery table, and a belt (9) for holding a body of the pregnant woman at a center portion thereof.

3. A delivery device as claimed in claim 1 or 2, characterized in that a roller (3) is disposed between the base (1) and the delivery table (2).

4. A delivery device as claimed in claim 1, 2 or 3, characterized by further comprising means for tilting the delivery device.

5. A delivery device, wherein a means (13) for giving an impact from a leg direction to a head direction of a delivery table (12) is disposed on a base (11) on which the delivery table is laid.

6. A delivery device as claimed in claim 5, characterized by further comprising a step (7) for placing a pregnant woman's leg for preventing the pregnant woman from slipping in the leg direction of the delivery table, and a belt (9) for holding a body of the pregnant woman at a center portion thereof.

7. A delivery device as claimed in claim 5 or 6, characterized in that the means (13) for giving impact is a rotary arm or an electric blow device.

8. A delivery device, wherein a delivery table (21) is arranged to oscillate in a longitudinal direction thereof and receives impact by colliding against a stopper (24) for a pregnant woman's legs so as to stimulate delivery.

9. A delivery device as claimed in claim 8, characterized by further comprising a handle (5) used for oscillating the delivery table (21), a step (7) for placing a pregnant woman's legs for preventing the pregnant woman from slipping in the leg direction of the delivery table, and a belt (9) for holding a body of the pregnant woman at a center portion thereof.
